# EUROPEAN PATENT APPLICATION

(11) **EP 3 906 863 A2**
(43) Date of publication of application: **10.11.2021**
(21) Application number: 21172059.4
(22) Date of filing: 04.05.2021
(51) Int. Cl.: A61B 17/12

(54) **DOUBLE LAYER BRAID FOR OCCLUSION OF ANEURYSMS**

(30) Priority: 04.05.2020 US 202016866102
(71) Applicant: DePuy Synthes Products, Inc., Raynham, MA 02767 (US)
(72) Inventor: GOROCHOW, Lacey, Raynham, 02767 (US)
(74) Representative: Carpmaels & Ransford LLP

(57) **Abstract**

A double layered braid for treating an aneurysm is provided. The braid can include a first radially expandable segment having a first plurality of wire segments with a first braid wire diameter. The braid can include a second radially expandable segment having a second plurality of wire segments with a second braid wire diameter, the second braid wire diameter being greater than the first braid wire diameter. The first segment can radially expand to form an outer occlusive sack that seals across a neck of the aneurysm. The second segment can radially expand within the outer occlusive sack to form an inner occlusive sack. The greater second braid wire diameter can allow the inner occlusive sack to exert a force against the outer occlusive sack and the walls of the aneurysm. The force can minimize movement of the outer occlusive sack from the neck of the aneurysm.

## Description

### Field of Invention

This disclosure relates to medical instruments, and more particularly, delivery systems for aneurysm therapy.

### Background

Aneurysms can be complicated and difficult to treat. For example, treatment access may be limited or unavailable when an aneurysm is located proximate critical tissues. Such factors are of concern with cranial aneurysms due to the brain tissue surrounding cranial vessels the corresponding limited treatment access.

Prior solutions have included endovascular treatment access whereby an internal volume of the aneurysm sac is removed or excluded from arterial blood pressure and flow. In this respect, because the interior walls of the aneurysm may continue being subjected to flow of blood and related pressure, aneurysm rupture remains possible.

Alternative to endovascular or other surgical approaches can include occlusive devices. Such devices have typically incorporated multiple embolic coils that are delivered to the vasculature using microcatheter delivery systems. For example, when treating cranial aneurysms, a delivery catheter with embolic coils is typically first inserted into non-cranial vasculature through a femoral artery in the hip or groin area. Thereafter, the catheter is guided to a location of interest within the cranium. The sac of the aneurysm can then be filled with the embolic material to create a thrombotic mass that protects the arterial walls from blood flow and related pressure. However, such occlusive devices do have certain shortcomings, including the fact that volume they can fill is somewhat permanent due to the thrombotic mass delivered therein.

One particular type of occlusive approach endeavors to deliver and treat the entrance or "neck" of the aneurysm as opposed to the volume of the aneurysm. In such "neck" approaches, by minimizing blood flow across the neck, then a venous stasis in the aneurysm may be achieved. In turn, a thrombotic mass may naturally form without having to deliver embolic materials, as previously described. This is preferable to masses formed from embolic material since a natural mass can improve healing by reducing possible distention from arterial walls and permits reintegration into the original parent vessel shape along the neck plane of the aneurysm. It is understood that the neck plane is an imaginary surface where the inner most layer of the parent wall would be but for the aneurysm. However, neck-occlusive approaches are not without drawbacks. It is typical for neck-occlusive approaches to fail to impede flow into blood vessels while also blocking the aneurysm neck in the parent vessel. This can unintentionally lead to severe damage if the openings of the vessels are blocked. Furthermore, embolic coils do not always effectively treat aneurysms as re-canalization of the aneurysm and/or coil compaction can occur over time.

It is therefore desirable to have a device which easily, accurately, and safely occludes a neck of an aneurysm or other arterio-venous malformation in a parent vessel without blocking flow into perforator vessels communicating with the parent vessel.

### Summary

It is an object of the present invention to provide systems, implants, and methods to meet the above-stated needs. Generally, it is an object of the present invention to provide an intrasaccular occlusive device for treating an aneurysm having a first radially expandable braided segment that expands to form an outer occlusive sack and a second radially expandable braided segment that expands to form an inner occlusive sack. The first and second radially expandable braided segments each include a plurality of wire segments. A majority of the wire segments of the second radially expandable segment have a greater braid wire diameter than a majority of the wire segments of the first radially expandable segment. When the occlusive device is in a deployed configuration within an aneurysm, the greater braid wire diameter of the wire segments of the first radially expandable segment allow the inner occlusive sack to exert a radially outward force against the outer occlusive sack and the walls of the aneurysm that minimizes movement of the outer occlusive sack.

An example occlusive device for treating an aneurysm can include a first radially expandable braided segment and a second radially expandable braided segment. The first radially expandable braided segment can be configured to form an outer occlusive sack in the aneurysm and can include a first plurality of wire segments. A majority of wire segments of the first plurality of wire segments can have a first braid wire diameter. The second radially expandable braided segment can be configured to form an inner occlusive sack in the aneurysm and can include a second plurality of wire segments. A majority of wire segments of the second plurality of wire segments can have a second braid wire diameter greater than the first braid wire diameter.

The first braid wire diameter can be between approximately 20 micrometers and approximately 25 micrometers.

The second braid wire diameter can be between approximately 25 micrometers and approximately 76 micrometers.

The first plurality of wire segments can include a greater number of wire segments than the first plurality of wire segments.

In the deployed configuration, the inner occlusive sack can provide a radially outward force against a wall of the aneurysm and the outer occlusive sack.

The outer occlusive sack can have a first open distal end and the inner occlusive sack can have a second open distal end.

The second open distal end can include a plurality of looped ends.

The first expandable braided segment and the second expandable braided segment can be coupled together proximally at a proximal end.

In the deployed configuration, the first expandable braided segment can expand radially to a substantially cylindrical first predetermined shape and the second expandable braided segment can expand radially to a substantially spherical second predetermined shape.

The first plurality of wire segments can have a first average braid wire diameter. The first average braid wire diameter can be a sum of the first braid wire diameter of each wire segment of the first plurality of wire segments divided by a total number of wire segments of the first plurality of wire segments.

The second plurality of wire segments can have a second average braid wire diameter. The second average braid wire diameter can be a sum of the second braid wire diameter of each wire segment of the second plurality of wire segments divided by a total number of wire segments of the second plurality of wire segments.

The second average braid wire diameter can be greater than the first average braid wire diameter.

The first average braid wire diameter can be between 20 micrometers and approximately 25 micrometers. The second average braid wire diameter can be between approximately 25 micrometers and approximately 76 micrometers.

Another example occlusive device for treating an aneurysm can include a first braid and a second braid. The first braid can be configured to form an outer occlusive sack in the aneurysm and include a first plurality of wire segments. A majority of wire segments of the first plurality of wire segments can have a first cross-section area. The second braid can be configured to form an inner occlusive sack in the aneurysm and include a second plurality of wire segments. A majority of wire segments of the second plurality of wire segments can have a second cross-section area greater than the first cross-section area.

The first cross-section area can be between approximately 3.2 x 10-4 millimeters squared and approximately 5.1 x 10-4 millimeters squared. The second cross-section area can be between approximately 5.1 x 10-4 millimeters squared and approximately 4.6 x 10-3 millimeters squared.

An example method of occluding an aneurysm can include one or more of the following steps presented in no particular order, and the method can include additional steps not included herein. The method can include delivering a first radially expandable braid including a first plurality of wire segments and a second radially expandable braid including a second plurality of wire segments through vasculature to the aneurysm. The first radially expandable braid and the second radially expandable braid can form a double layered braid coupled together at a proximal end.

The method can include distally pushing the double layered braid into the aneurysm whereby the second radially expandable braid expands to form an inner occlusive sack.

The method can include further distally pushing the double layered braid thereby expanding the first radially expandable braid to form an outer occlusive sack surrounding the inner occlusive sack.

The method can include positioning the second radially expandable braid in communication with a wall of the aneurysm and the outer occlusive sack such that the second radially expandable braid can exert a force against the wall of the aneurysm and the outer occlusive sack. The force can minimize movement of the first radially expandable braid.

The method can include defecting, diverting, or slowing flow into the aneurysm across the neck of the aneurysm when the outer occlusive sack is formed across the neck and the inner occlusive sack is formed within the outer occlusive sack.

A majority of wire segments in the first plurality of wire segments can include a first braid wire diameter that is less than a second braid wire diameter of a majority of the wire segments in the second plurality of wire segments.

The first plurality of wire segments can have a first average braid wire diameter and the second plurality of wire segments can have a second average braid wire diameter. The first average braid wire diameter can be less than the second average braid wire diameter.

Each of the wire segments in the first plurality of wire segments can have a first cross-section area and each of the wire segments in the second plurality of wire segments can have a second cross-section area. The first cross-section area can be less than the second cross-section area.

The method can include expanding the second radially expandable braid within the outer occlusive sack while the outer occlusive sack is pushed against a wall of the aneurysm and a neck of the aneurysm.

### Brief Description of the Drawings

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1A depicts an example occlusive device in a collapsed configuration, according to aspects of the present invention;
FIG. 1B depicts an example occlusive device in a collapsed configuration within an example microcatheter, according to aspects of the present invention;
FIG. 2 depicts an example occlusive device, wherein the occlusive device is being deployed, according to aspects of the present invention;
FIG. 3 is a schematic side view of an example delivery system with an occlusive device in a deployed configuration, according to aspects of the present invention;
FIG. 4A illustrates a first plurality of wires having a first braid wire diameter, according to aspects of the present invention;
FIG. 4B illustrates a first plurality of wires having a first cross-section area, according to aspects of the present invention;
FIG. 5A illustrates a second plurality of wires having a second braid wire diameter, according to aspects of the present invention;
FIG. 5B illustrates a second plurality of wires having a second cross-section area, according to aspects of the present invention;
FIG. 6A illustrates an example double layered braid prior to being configured into a collapsed configuration, according to aspects of the present invention;
FIG. 6B illustrates the example double layered braid of FIG. 6A in a collapsed configuration, according to aspects of the present invention;
FIG. 7A-7D are a sequence of perspective schematic side views depicting steps of deploying the occlusive device of FIGs. 6A and 6B into an aneurysm, wherein the occlusive device is shown moving from a collapsed configuration to a deployed configuration, according to aspects of the present invention;
FIG. 8A illustrates an additional example double layered braid configured into a collapsed configuration, according to aspects of the present invention;
FIG. 8B illustrates a cross-section of the additional example double layered braid of FIG. 8A, according to aspects of the present invention;
FIG. 8C illustrates the additional example double layered braid of FIG. 8A in a deployed configuration, according to aspects of the present invention;
FIG. 9A-9D are a sequence of illustrations depicting steps of deploying the example occlusive device of FIGs. 8A through 8C into an aneurysm, wherein the occlusive device is shown moving from a collapsed configuration to a deployed configuration, according to aspects of the present invention;
FIG. 10A is a perspective schematic view showing an example delivery system for use with an example occlusive device, according to aspects of the present invention;
FIG. 10B is a perspective schematic view of FIG. 10A but with a partial cross-section of the delivery system and the occlusive device, according to aspects of the present invention;
FIG. 11A is a perspective schematic view of FIGs. 10A-10B being deployed with a partial cross-section of the delivery system and the occlusive device, according to aspects of the present invention;
FIG. 11B is a perspective schematic view of FIGs. 10A-10B deployed with the example delivery system detached from the occlusive device, according to aspects of the present invention;
FIGs. 12A-12D depict examples of a double layered braid, according to aspects of the present invention; and
FIG. 13 is a flow diagram for a method of occluding an aneurysm, according to aspects of the present invention.

### Detailed Description

A double layered braid for treating an aneurysm is provided. The braid can include a first radially expandable segment having a first plurality of wire segments with a first braid wire diameter. The braid can also include a second radially expandable segment having a second plurality of wire segments with a second braid wire diameter, the second braid wire diameter being greater than the first braid wire diameter. The first segment can radially expand to form an outer occlusive sack that seals across a neck of the aneurysm. The second segment can radially expand within the outer occlusive sack to form an inner occlusive sack. The greater braid wire diameter of the second plurality of wire segments that can form inner occlusive sack can exert a force against the outer occlusive sack and the walls of the aneurysm. The force can minimize movement of the outer occlusive sack from the neck of the aneurysm.

Although example embodiments of the disclosed technology are explained in detail herein, it is to be understood that other embodiments are contemplated. Accordingly, it is not intended that the disclosed technology be limited in its scope to the details of construction and arrangement of components set forth in the following description or illustrated in the drawings. The disclosed technology is capable of other embodiments and of being practiced or carried out in various ways.

It must also be noted that, as used in the specification and the appended claims, the singular forms "a," "an" and "the" include plural referents unless the context clearly dictates otherwise. By "comprising" or "containing" or "including" it is meant that at least the named compound, element, particle, or method step is present in the composition or article or method, but does not exclude the presence of other compounds, materials, particles, method steps, even if the other such compounds, material, particles, method steps have the same function as what is named.

In describing example embodiments, terminology will be resorted to for the sake of clarity. It is intended that each term contemplates its broadest meaning as understood by those skilled in the art and includes all technical equivalents that operate in a similar manner to accomplish a similar purpose. It is also to be understood that the mention of one or more steps of a method does not preclude the presence of additional method steps or intervening method steps between those steps expressly identified. Steps of a method may be performed in a different order than those described herein without departing from the scope of the disclosed technology. Similarly, it is also to be understood that the mention of one or more components in a device or system does not preclude the presence of additional components or intervening components between those components expressly identified.

As discussed herein, vasculature can be that of any "subject" or "patient" including of any human or animal. It should be appreciated that an animal may be a variety of any applicable type, including, but not limited thereto, mammal, veterinarian animal, livestock animal or pet type animal, etc. As an example, the animal may be a laboratory animal specifically selected to have certain characteristics similar to a human (e.g., rat, dog, pig, monkey, or the like). It should be appreciated that the subject may be any applicable human patient, for example.

As discussed herein, "operator" may include a doctor, surgeon, or any other individual or delivery instrumentation associated with delivery of a braid body to the vasculature of a subject.

As discussed herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

Cerebrovascular aneurysms are known to be treated using embolic coils, which are delivered to the aneurysm sack via a microcatheter and detached in situ. It is understood that "packing density" is the volume of the aneurysm sack occupied by the coil mass. In previous coil approaches, multiple coils (e.g. five coils) have been used to pack the aneurysms and the packing density can typically range between 20-25%. The herein disclosed device improves on prior approaches by being operable to seal the aneurysm neck and pack the aneurysm to a higher packing density while avoiding risk of aneurysm rupture during package.

In previous embolic-based approaches, packing the aneurysm required in placement of coils into the aneurysm sack until the aneurysm obtained the desired packing density to occlude the aneurysm. However, obtaining such a density without risk of rupture was difficult, unsafe, and aneurysm morphology (e.g. wide neck, bifurcation, etc.), and the like, rendered it difficult, if not impossible, for an operator to re-position the coils once delivered and installed on site. Furthermore, aneurysms treated with multiple coils often reanalyze or compact as a result of poor coiling, lack of coverage across the aneurysm neck, as a result of flow, or even aneurysm size.

Relatedly, flow diverters that are deployed across the aneurysm neck can alter the flow of blood into the aneurysm. An example flow diverter can be a braided device with relatively low porosity. Over time, the aneurysms can heal by sealing the aneurysm neck with a high rate of success. However, flow diversion technology is not without limitations. Challenges include placement of the devices intra-vascularly due to vessel morphology, vessel tortuosity, or braid malposition. In addition, patients receiving a flow diverter must be on anticoagulation medicine for an extended period to prevent vessel thrombosis. Intrasaccular devices also aim to cut circulation into the aneurysm while minimizing the amount of metal in the vessel and significantly cutting, or eliminating the need for coagulation medication. These types of devices may also be easier to track and/or deploy at the lesion site.

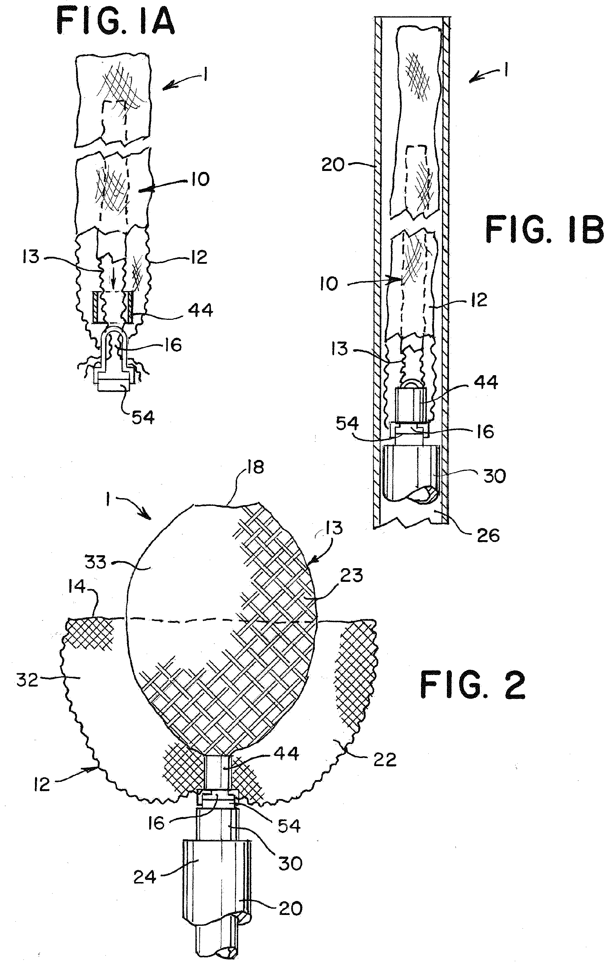

The occlusive device 1 disclosed herein addresses these and other drawbacks of previous approaches. Turning to FIG. 1A, an example occlusive device 1 of this disclosure is shown in a collapsed configuration prior to being arranged with a microcatheter 20. FIG. 1B depicts the occlusive device 1 of FIG. 1A arranged in the collapsed configuration within the microcatheter 20. As shown, device 1 can include a double layered braid 10 formed from multiple self-expanding segments 12, 13 that can be formed from a plurality of wire segments 32, 33. In a deployed configuration, the doubled layered braid 10 can include a first radially expandable braided segment 12 associated with an outer occlusive sack 22 and a second radially expandable braided segment 13 associated with an inner occlusive sack 23. The first radially expandable braided segment 12 and the second radially expandable segment 13 can be coupled together at a proximal end 16.

As shown in FIGs. 1B, FIG. 2, and FIG. 3, a delivery tube 30 can be slidably disposed within the microcatheter 20. The microcatheter 20 can be pre-placed at the level of the neck 104 of the aneurysm A and used to track the device 1 to the aneurysm A. The microcatheter 20 size can be selected in consideration of the size, shape, and directionality of the aneurysm A or features through which the microcatheter 20 can pass to get to the treatment site. The microcatheter 20 can have a total usable length anywhere from 80 centimeters to 170 centimeters. The microcatheter 20 can have an inner diameter of anywhere between 0.015 and 0.032 inches. The outer diameter can also range in size and may narrow at either its proximal end 26 or distal end 24. At its proximal end 26, the microcatheter 20 can be attached to a surgical device, and at its distal end 24 may be operable to positioned at the neck 104 of the aneurysm A. While the distal end 24 of the microcatheter 20 as shown contains the braid 10, the distal end 24 can be varied in shape and can curve at an angle.

The delivery tube 30 can be substantially elongated and can extend from the proximal end 26 to the distal end 24 of microcatheter 20. The delivery tube 30 can generally run along the inner lumen of microcatheter 20 and can leave a space between its outer surface and the internal surface of microcatheter 20. In turn, the delivery tube 30 and the microcatheter 30 can be axially aligned. The delivery tube 30 can deliver the braid 10 to a location of interest (e.g. a lesion site) using microcatheter 20. In certain examples, the microcatheter 20 can be pre-placed at a level of the neck 104 of the aneurysm A and used to track the device 1 to the lesion, for example by tracking marker band 44. The delivery tube 30 can be in mechanical connection with the braid 10 at locking portion 54. As shown more particularly below, the locking portion 54 can comprise or be a pusher ring. The braid 10 can be attached to locking portion 54 by slidable attachment, permanent attachment (e.g. crimped, laser, ultrasonic weld, or other sources of heat, adhesive, or the like) or other attachment approaches. When delivery tube 30 is mechanically attached to braid 10 at locking portion 54, distally translating, sliding, or otherwise moving tube 30 towards the aneurysm A can cause braid 10 to begin moving from the collapsed configuration within microcatheter 20 to its deployed configuration external to microcatheter 20 with segments 12 and 13.

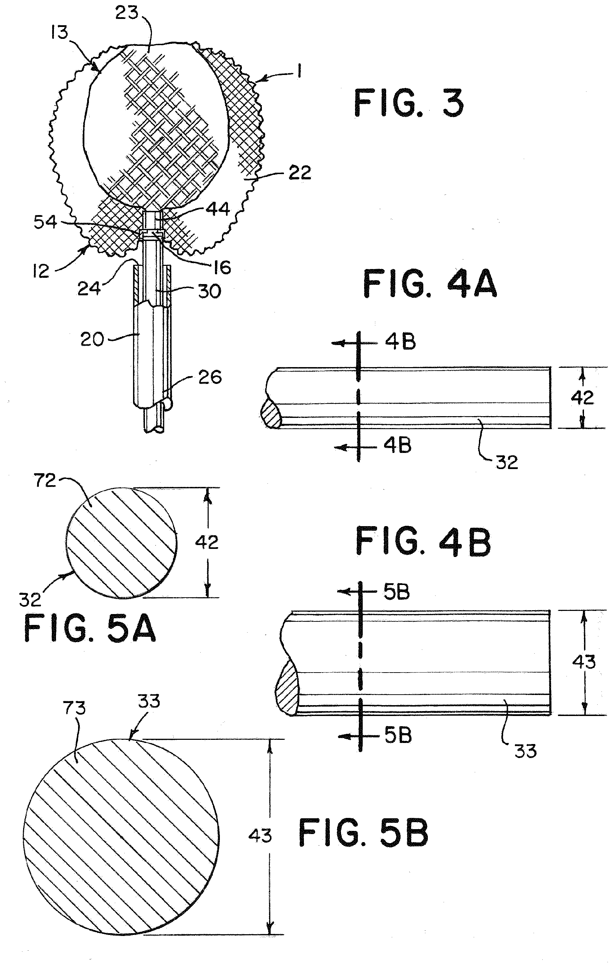

FIGs. 2 and 3 illustrate the example occlusive device 1 in a deployed configuration. In the deployed configuration, some or all of braid 10 is distal of microcatheter 20 so that segments 12, 13 can radially expand. Braid 10 is particularly advantageous as it is capable of being collapsed within microcatheter 20 while also being capable of forming multiple occlusive sacks 22, 23 in the deployed configuration. As braid 10 is distally translated, segment 13 can begin to radially expand to form the inner occlusive sack 23. As braid 10 is further distally translated, segment 12 can begin to radially expand external to the inner occlusive sack 23 and form the outer occlusive sack 22. As illustrated in FIGs. 2 and 3, in the deployed configuration, the first and second radially expandable segments 12, 13 can still be connected to delivery tube 30 via locking portion 54 at the proximal end 16.

In the deployed configuration, a first distal end 14 of the first radially expandable segment 12 can be open such that the second radially expandable segment 13 can internally expand within the outer occlusive sack 22. A second distal end 18 of the second radially expandable segment 13 can also be open. In one example, in the deployed configuration, the distal end 18 of the second segment 13 can have a smaller diameter than the distal end 14 of the first segment 12, as the inner occlusive sack 23 can be further distally pushed into the dome of the aneurysm A.

The first radially expandable segment 12 can comprise a first plurality of wire segments 32 and the second radially expandable segment 13 can comprise a second plurality of wire segments 33. The wire segments can be made from nitinol with interwoven platinum filaments for radiopacity or Drawn Filled Tube (DFT) Nitinol with 10 to 40% Platinum. The wire segments can be made from a nickel-titanium alloy, cobalt chromium alloys, Stainless Steel, Tantalum, and/or other alloys, and/or any other suitable biocompatible materials, or combination of these materials. Also, these materials can be absorbable or non-absorbable by the patient over time. The first and second plurality of wire segments 32,33 can form one or more meshes of braid 10. The mesh of braid 10 can be configured so that as braid 10 is distally translated and the first open distal end 14 exits from within microcatheter 20, segment 12 can radially expand to form an outer occlusive sack 22. The outer occlusive sack 22 can be formed as distal end 14 slides away from distal end 24 of microcatheter 20.

Turning to FIG. 3, an enlarged schematic side view of the braid 10 as illustrated in FIG. 2 is shown in a close-up, deployed configuration but not delivered to an aneurysm A. As shown, each of segment 13 can have a generally spherical shaped segment associated with its respective inner occlusive sack 23, while segment 12 can have portions about and/or in communication with marker band 44 that can be mirrored ellipsoids. Although FIGs. 2 and 3 illustrate two different examples of shapes the outer occlusive sack 22 and the inner occlusive sack 23 can have when the braid 10 is in a deployed configuration, it is contemplated that the outer occlusive sack 22 and the inner occlusive sack 23 can have a variety of shapes, including spherical, saddled, cylindrical, tubular, ellipsoid, or any other shape.

FIGs. 4A through 5B illustrate the differing diameters and cross-section areas of the first plurality of wire segments 32 and the second plurality of wire segments 33. Each wire segment within the first plurality of wire segments 32 can have a first braid wire diameter 42 and each wire segment within the second plurality of wires 33 can have a second braid wire diameter 43. The first braid wire diameter 42 for a majority of the wire segments in the first plurality of wire segments 32 can be smaller than the second braid wire diameter 43 for a majority of the wire segments in the second plurality of wire segments 33. In one example, each of the wire segments in the first plurality of wire segments 32 can have a smaller braid wire diameter than each of the wire segments in the second plurality of wire segments 33. Further, the first plurality of wire segments 32 can have an average first braid wire diameter. The average first braid wire diameter can be the sum of the first braid wire diameter 42 for each of the wire segments in the first plurality of wire segments 32 divided by the total number of wire segments in the first plurality of wire segments 32. Similarly, the second plurality of wire segments 33 can have an average second braid wire diameter. The average second braid wire diameter can the sum of the second braid wire diameter 43 for each of the wire segments in the second plurality of wire segments 33 divided by the total number of wire segments in the second plurality of wire segments 33. The average first braid wire diameter can be smaller than the average second braid wire diameter.

In one example, the first braid wire diameter 42 for a majority of wire segments of the first plurality of wire segments 32 can be between approximately 20 micrometers and approximately 35 micrometers. The second dimeter 43 for a majority of wire segments of the second plurality of wire segments 33 can be between approximately 25 micrometers and approximately 76 micrometers.

The first plurality of wire segments 32 can include a greater number of wire segments than the second plurality of wire segments 33. In one example, the first plurality of wire segments 32 can include between approximately 40 and approximately 100 wire segments and the second plurality of wire segments 33 can include between approximately 10 and approximately 40 wire segments.

FIGs. 4B and 5B illustrate cross-section views of a wire segment in the first plurality of wire segments 32 and the second plurality of wire segments 33. In FIGs. 4B and 5B, the wire segments are shown with circular cross-section areas; however, it is contemplated that the wire segments can have a cross-section of a plurality of shapes, including but not limited to, cuboid, ellipsoid, triangular, flat, and the like. The cross-section area 72 of the first plurality of wire segments 32 can be smaller than the cross-section area 73 of the second plurality of wire segments 33. In one example, the cross-section area 72 of the first plurality of wire segments 32 can be between approximately 3.2 x 10-4 millimeters squared and approximately 5.1 x 10-4 millimeters squared. The cross-sectional area 73 of the second plurality of wire segments 33 can be between approximately 5.1 x 10-4 millimeters squared and approximately 4.6 x 10-3 millimeters squared.

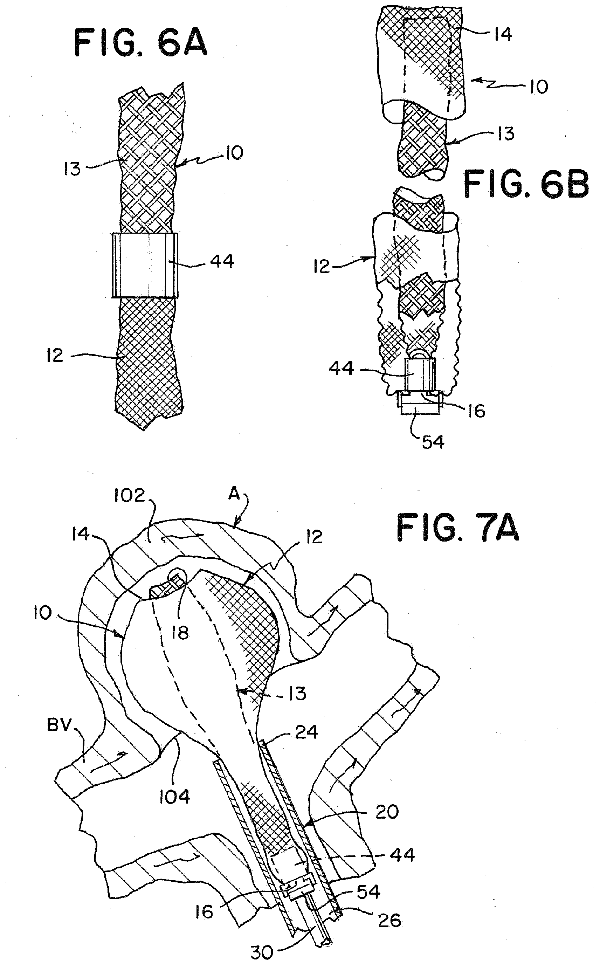

FIG. 6A illustrates an example double layered braid 10 prior to the braid 10 being configured in a collapsed configuration. The double layered braid 10 can include the first radially expandable braided segment 12 and the second radially expandable braided segment 13. The first radially expandable segment 12 can include the first plurality of wire segments 32 having a first braid wire diameter 42. The second radially expandable segment 13 can include the second plurality of wire segments 33 having a second braid wire diameter 43 that is greater than the first braid wire diameter 42 of the wire segments of the first segment 12.

In one example, the doubled layered braid 10 can be formed from two separate and distinct meshes, where a first mesh comprises the first radially expandable segment 12 including wire segments with a first braid wire diameter 42, and a second mesh comprises the second radially expandable segment 13 including wire segments with a second braid wire diameter 43, the second braid wire diameter being greater than the first braid wire diameter. Alternatively, the double layered braid 10 can be formed from a single mesh having a first region and a second region. The first region can correspond to the first radially expandable segment 12 and the second region can correspond to the second radially expandable segment 13. The first region can include wire segments having a first braid wire diameter 42 and the second region can include wire segments having a second braid wire diameter 43, the second braid wire diameter 43 being greater than the first braid wire diameter 42.

FIG. 6B illustrates the double layered braid 10 in the collapsed configuration. The proximal end 16 can be disposed on or adjacent to the marker band 44 and the locking portion 54. The first open distal end 14 can be inserted through marker band 44 until the proximal end 16 is disposed on or adjacent to the marker band 4 at the locking portion 54. The locking portion 54 can then be connected to and/or folded over the proximal end 16. The proximal end 16 of the braid can be operatively connected to locking portion 54 by sonic weld, mechanical attachment, or adhesive.

FIGs. 7A through 7D are a sequence of illustrations depicting pushing the occlusive device 1 into an aneurysm A thereby allowing the segments 12, 13 to radially expand and form an outer occlusive sack 22 and an inner occlusive sack 23, respectively. In particular, FIGs. 7A through 7D depict an enlarged schematic side view of a delivery system, including the delivery tube 30 and the microcatheter 20, and double layered braid 10 as the braid 10 is being pushed into the aneurysm A. Prior to the arrangement of FIG. 7A, the braid 10 can be assembled with a delivery tube 30 and/or a microcatheter 20 in a collapsed configuration, as disclosed herein. In this respect, the delivery system and the braid 10 can be packaged as a portable kit or system. The assembly between microcatheter 20, delivery tube 30, and/or braid 10 can take place before being introduced into the vasculature. The delivery system used with braid 10 can be selectively positioned at the lesion site and delivery tube 30 can begin distally translating braid 10 towards the aneurysm A. When the braid 10 is initially translated towards the aneurysm A, the segment 12 can be a generally spherical shape internal to aneurysm A while segment 13 in turn remains mostly collapsed and stored within microcatheter 20. However, a portion of segment 13 distal of the microcatheter 20 on or about the distal end 18 of segment 13 can begin to radially expand. Delivery tube 30 can include one or more fasteners 54 operable to securely fasten braid 10 in place prior to deployment.

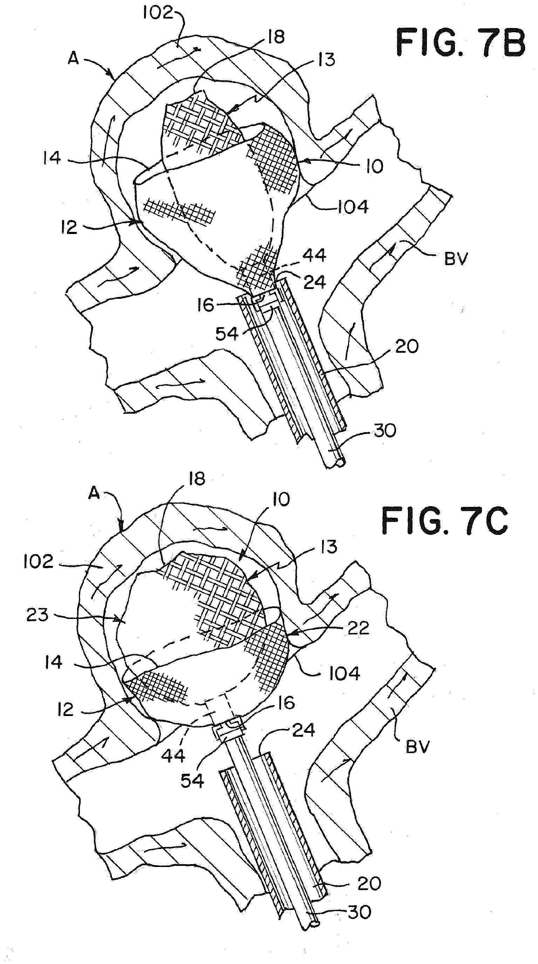

In FIG. 7B, the delivery tube 30 can distally slide braid 10 deeper into aneurysm A. The marker band 44 can be distally translated closer and tucked into the neck 104 of aneurysm A. It is understood that the outer surface of braid 10 can be made from nitinol with interwoven platinum filaments for radiopacity. Delivery tube 30 may be driven by a hypotube from its proximal end 36 (not depicted), by an operator, or the like. Microcatheter 20 may remain relatively stationary or fixed while delivery tube 30 can be seen distally translating braid 10 towards and through the neck 104 of aneurysm A. Further translating braid 10 into aneurysm A, as is shown in FIG. 7B can cause the inner occlusive sack 23 of segment 13 to continue to form inside of the outer occlusive sack 22.

FIG. 7C illustrates the delivery tube 30 can distally slide braid 10 deeper into aneurysm A allowing first segment 12 and second segment 13 to continue to radially expand to form the outer occlusive sack 22 and the inner occlusive sack 23, respectively. In certain embodiments, the widening of segment 12 between FIGs. 7A and 7A can cause the first open distal end 14 to slide proximally back towards the distal end 24 of microcatheter 20 while segment 13 continues to expand radially. For example, as the first open distal end 14 of segment 12 expands to a larger diameter between FIGs. 7A and 7A, the distal end 14 can also be drawn proximally from the second distal end 18 yet expand outwardly while the second open distal end 18 can remain on or adjacent the dome of the aneurysm A.

As also seen moving between FIGs. 7A to 7C, the junction between the proximal end 16 of braid 10, locking portion 54, and delivery tube 30 can move from within microcatheter 20 in the collapsed configuration to completely within aneurysm A in the deployed configuration. Once braid 10, including segments 12 and 13, are selectively positioned and arranged to the desired condition (e.g. braid 10 has been translated distally to expand segments 12, 13 to form the outer and inner sacks, respectively) the outer occlusive sack 22 of segment 12 can be seen being sealed against the neck 104 of the aneurysm A to deflect, divert or slow flow into the aneurysm A. The larger second braid wire diameter 43 of a majority of the wire segments in the second plurality of wire segments 33 that make up the second segment 13 allow the inner occlusive sack 23 to exert a force against the walls 102 of the aneurysm and the outer occlusive sack 22. This exerted force can advantageously prevent the outer occlusive sack 23 from moving or sliding from the neck 104 of the aneurysm A.

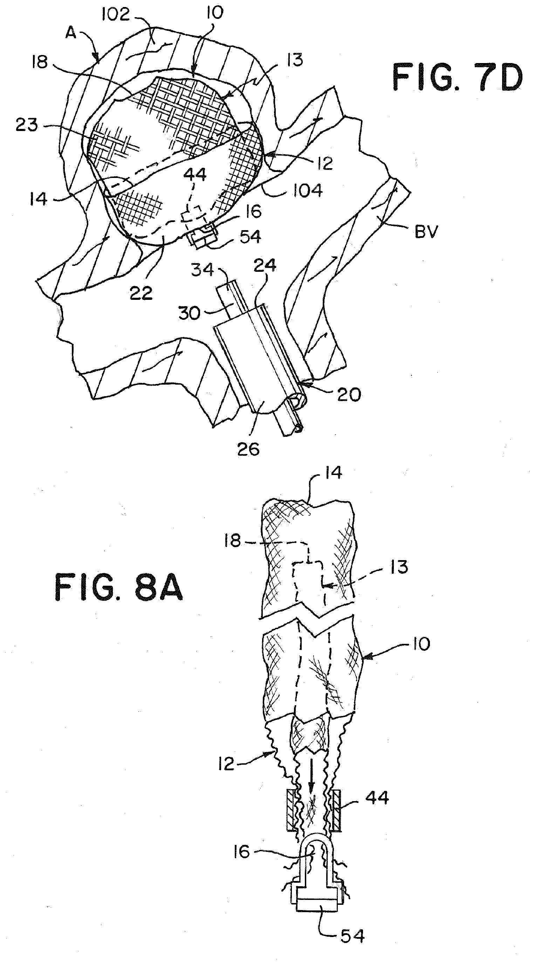

In FIG. 7D, when the braid 10 is in its fully deployed configuration and properly positioned within the aneurysm A, the braid 10 can be detached from the delivery tube 30. The implanted double layered braid 10 can remain in the aneurysm A and effectively deflect, divert, or slow flow into the aneurysm A. However, if either of the outer occlusive sack 22 or inner occlusive sack 23 of segments 12, 13, respectively, is not precisely positioned or need to be reset or adjusted within aneurysm A for safe occlusion without risk of rupture, braid 10 can be retracted back into microcatheter 20 by proximally withdrawing delivery tube 30 while still attached to braid 10.

FIGs. 8A illustrates an additional example double layered braid 10 in a collapsed configuration. The double layered braid 10 can include a first expandable segment 12 and a second expandable segment 13. The doubled layered braid 10 can be formed from two separate and distinct meshes, where a first mesh comprises the first radially expandable segment 12 including wire segments with a first braid wire diameter 42, and a second mesh comprises the second radially expandable segment 13 including wire segments with a second braid wire diameter 43, the second braid wire diameter being greater than the first braid wire diameter. Alternatively, the double layered braid 10 can be formed from a single mesh having a first region and a second region. The first region can correspond to the first radially expandable segment 12 and the second region can correspond to the second radially expandable segment 13. The first region can include wire segments having a first braid wire diameter 42 and the second region can include wire segments having a second braid wire diameter 43, the second braid wire diameter 43 being greater than the first braid wire diameter 42.

The first expandable segment can have an open first distal end 14 and the second expandable segment can have an open second distal end 18. The first expandable segment 12 and the second expandable segment 13 can be affixed together at a proximal end 16. In one example, the first expandable segment 12 and the second expandable segment 13 can be crimped, pinched, ultrasonic welded, or otherwise affixed together at the proximal end 16. The first expandable segment 12 and the second expandable segment 13 can be affixed to the marker band at the proximal end 16. The proximal end 16 can be disposed on or adjacent to the marker band 44 and the locking portion 54. The braid 10 can be attached to locking portion 54. When delivery tube 30 is mechanically attached to braid 10 at locking portion 54, distally translating, sliding, or otherwise moving tube 30 towards the aneurysm A can cause braid 10 to begin moving from the collapsed configuration within microcatheter 20 to its deployed configuration external to microcatheter 20 with segments 12 and 13.

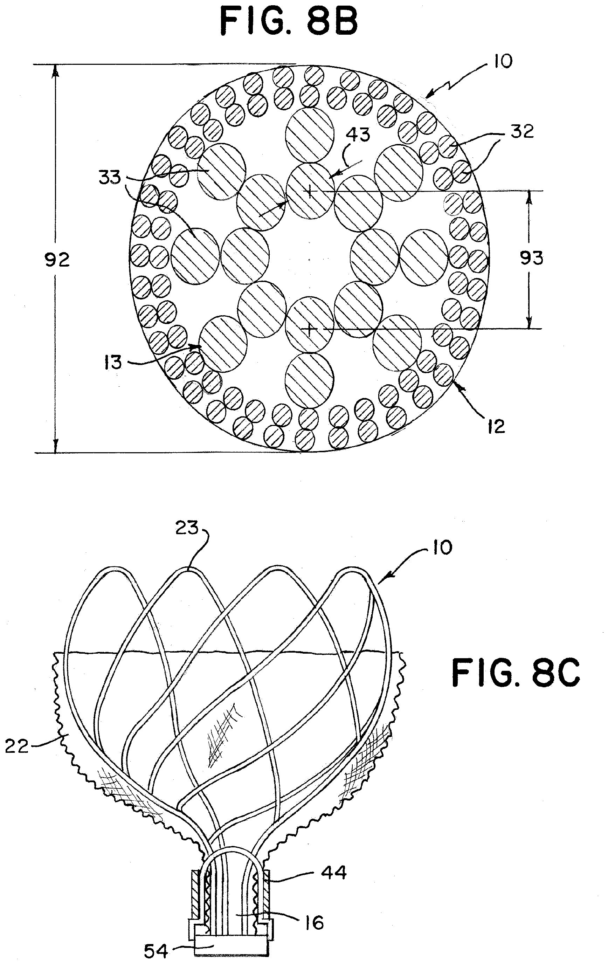

FIG. 8B illustrates a cross-section view of the double layered braid 10 in the collapsed configuration. The first expandable segment 12 can have an outer diameter 92 and the second expandable segment 13 can have an inner diameter 93. When the double layered braid 10 is configured in the collapsed configuration, the first plurality of wire segments 32 of the first expandable segment 12 can overlap with each other and form two layers of wire segments. Similarly, the second plurality of wire segments 33 of the second expandable segment 13 can overlap with each other and form two layers of wire segments. The wire segments of the first plurality of wire segments 32 can have a first braid wire diameter 42 that is smaller than the second braid wire diameter 43 of the wire segments in the second plurality of wire segments 33. The second expandable segment 13 can have fewer wire segments than the first expandable segment 12. By way of example, the first plurality of wire segments 32 can include approximately 72 wire segments and each wire segment can have a first braid wire diameter 42 of approximately 0.20 millimeters (0.0008 inches). The second plurality of wire segments 33 can include approximately 16 wire segments and each wire segment can have a second braid wire diameter of approximately 0.05 millimeters (0.002 inches). In this configuration, the double layered braid 10 can include a total of 88 wire segments and the average braid wire diameter can be approximately 0.02 millimeters (0.001 inches). By having fewer wire segments, the second expandable segment 13 can collapse to fit within the first expandable segment 12 when the first expandable segment 12 is collapsed to the outer diameter 92. The second expandable segment 13 can have a smaller inner diameter 93 than the outer diameter 92 of the first expandable segment 12. By way of example, the outer diameter 92 of the first segment 12 can be approximately 0.35 millimeters (0.014 inches) and the inner diameter 93 of the second segment 13 can be approximately 0.08 millimeters (0.003 inches), allowing the second segment 13 to be disposed within the first segment 12 in the collapsed configuration.

This configuration of the double layered braid 10 can provide advantages compared to a single layered braid. In the collapsed configuration, the single layered braid can define a hollow tube surrounded by two layers of wire segments. As illustrated in FIG. 8B, in the collapsed configuration, the double layered braid 10, the first expandable segment 12 can form two outer layers of wire segments and the second expandable segment 13 can form two inner layers of wire segments disposed within the hollow tube formed by the first expandable segment 12, where the wire segments of the first segment 12 have a first diameter 43 that is smaller than the second diameter 43 of the wire segments of the second expandable segment 13. A single layered braid including the same amount of wire segments and a same average braid wire diameter as a double layered braid 10 would necessarily have a greater outer diameter. By way of example, a single layered braid having eighty-eight (88) wire segments and an average braid wire diameter of approximately 0.02 millimeters (0.001 inches) would have an outer diameter of approximately 0.43 millimeters (0.017 inches). Thus, a microcatheter with an inner diameter of at least 0.43 millimeters would be necessary in order to receive the single layered braid. In contrast as discussed herein, the double layered braid 10 having the same amount of wire segments and same average braid wire diameter would have an outer diameter 92 of approximately 0.35 millimeters. Thus, a microcatheter 20 with a smaller inner diameter can receive the double layered braid 10, as compared to the microcatheter necessary to receive a single layered braid. A microcatheter 20 with a relatively smaller inner diameter can facilitate tracking the occlusive device 1 through the tortuous path of vasculature.

FIG. 8C illustrates the double layered braid 10 of FIG. 8A in a deployed configuration. In the deployed configuration, the first expandable segment 12 can radially expand to form an outer occlusive sack 22. The second expandable segment 13 can radially expand to form an inner occlusive sack 23 disposed within the outer occlusive sack 22. In the deployed configuration, the outer occlusive sack 22 and the inner occlusive sack 23 can remain affixed together at a proximal end 16. The proximal end 16 can remain disposed on or adjacent to the marker band 44 and the locking portion 54. In the deployed configuration, the greater second braid wire diameter 43 of the second plurality of wire segments 33 can allow the inner occlusive sack 23 to exert a force against the outer occlusive sack 22 and the wall 102 of the aneurysm A. The exerted force can prevent the outer occlusive sack 23 positioned to seal across a neck 104 of the aneurysm A from sliding, slipping, or otherwise moving away from the neck 104 of the aneurysm A. The outer occlusive sack 23 can include a greater number of wire segments than the inner occlusive sack 22. Although a majority of the wire segments of the first plurality of wire segments 32 of the outer occlusive sack 22 can have a smaller first braid wire diameter 42 than a majority of the wire segments of the second plurality of wire segments 33 of the inner sack 23, the increased number of wire segments of the outer sack 23 can provide adequate coverage and stability to the double layered braid 10.

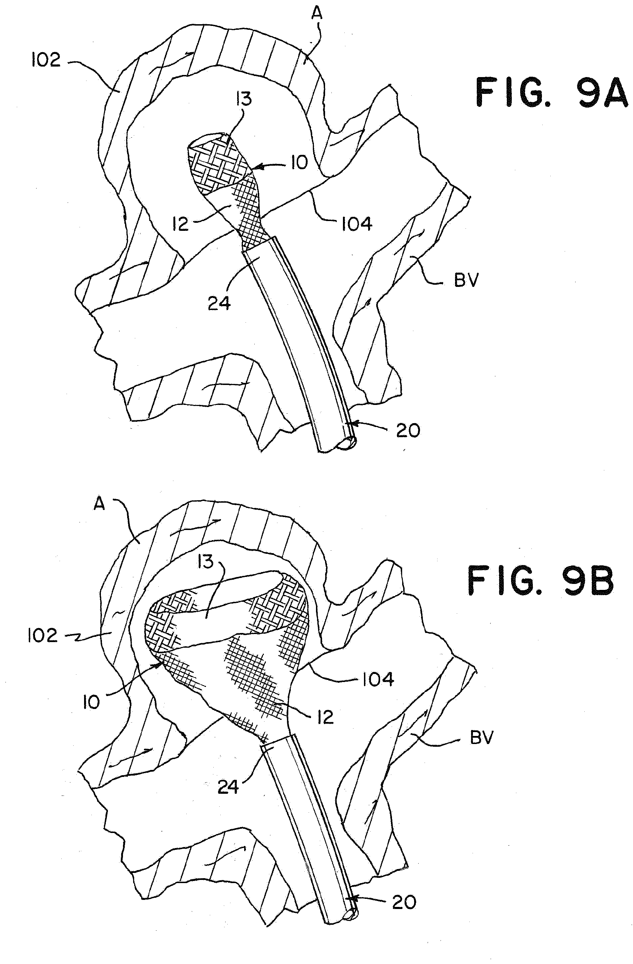

FIGs. 9A through 9D depict examples of the double layered braid 10 as illustrated in FIGs. 8A and 8B being deployed and delivered to an aneurysm A within a blood vessel BV as discussed previously herein. FIG. 9A illustrates braid 10 being initially advanced within the aneurysm A. The distal end 24 of the microcatheter 20 can be selectively positioned at the neck 104 of the aneurysm A. The second radially expandable segment 13 can be distally advanced within the aneurysm A. The first radially expandable segment 12 can be distally advanced within the aneurysm A such that the segment 12 can surround segment 13.

FIG. 9B illustrates the braid 10 being further distally advanced within the aneurysm A. As the braid 10 is distally pushed into the aneurysm A, the first expandable segment 12 and the second expandable segment 13 can continue to radially expand.

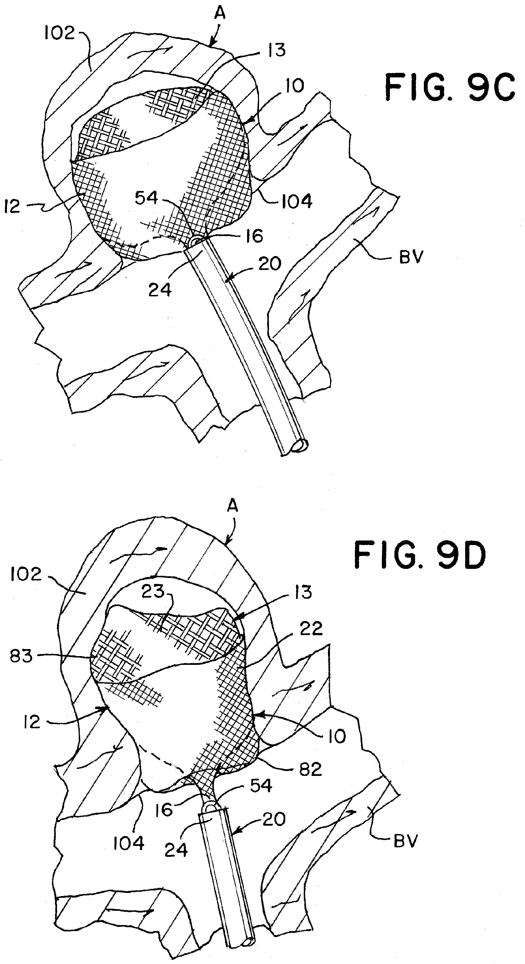

FIG. 9C illustrates the first expandable segment 12 can begin to radially expand to form the outer occlusive sack 22. Within the outer occlusive sack 22, the second expandable segment 13 can begin to radially expand to form the inner occlusive sack 23. The outer occlusive sack 22 can be positioned within the neck 104 of the aneurysm A to begin creating a seal against the neck 104.

FIG. 9D illustrates the braid 10 in the fully deployed configuration. In the deployed configuration, the outer occlusive sack 22 and the inner occlusive sack 23 can be formed. As illustrated in FIGs. 9C and 9D, the inner occlusive sack 23 can extend past the outer occlusive sack 22. In this configuration, the second open distal end 18 can be positioned farther into the dome of the aneurysm A. When the braid 10 is deployed within an aneurysm A, the outer occlusive sack 22 and the inner occlusive sack 23 can have a first predetermined shape 82 and a second predetermined shape 83, respectively. The first predetermined shape 82 and the second predetermined shape 83 can be confined by the anatomy of the aneurysm A in which the braid 10 is implanted. The outer occlusive sack 22 can radially expand to form a substantially cylindrical first predetermined shape 82. The cylindrical first predetermined shape 82 can effectively seal the neck 104 of the aneurysm A, and thus, deflect, divert, or slow flow to the aneurysm A. The inner occlusive sack 23 can radially expand to form a substantially spherical second predetermined shape 83. The greater second braid wire diameter 43 of the second plurality of wire segments 33 that form the inner occlusive sack 23 can exert a substantial force against the outer occlusive sack 23 and the walls 102 of the aneurysm A. This force can minimize movement of the outer occlusive sack 22 away from the neck 104 of the aneurysm A.

Although the example double layered braid 10 disclosed herein illustrates two occlusive sacks, it is contemplated that any number of internal occlusive sacks can be positioned internal to segment 12 and the one or more additional inner occlusive sacks can be formed as braid 10 is distally translated into the aneurysm A. The additional internal occlusive sacks can be formed from one or more additional segments capable of forming inner occlusive sacks.

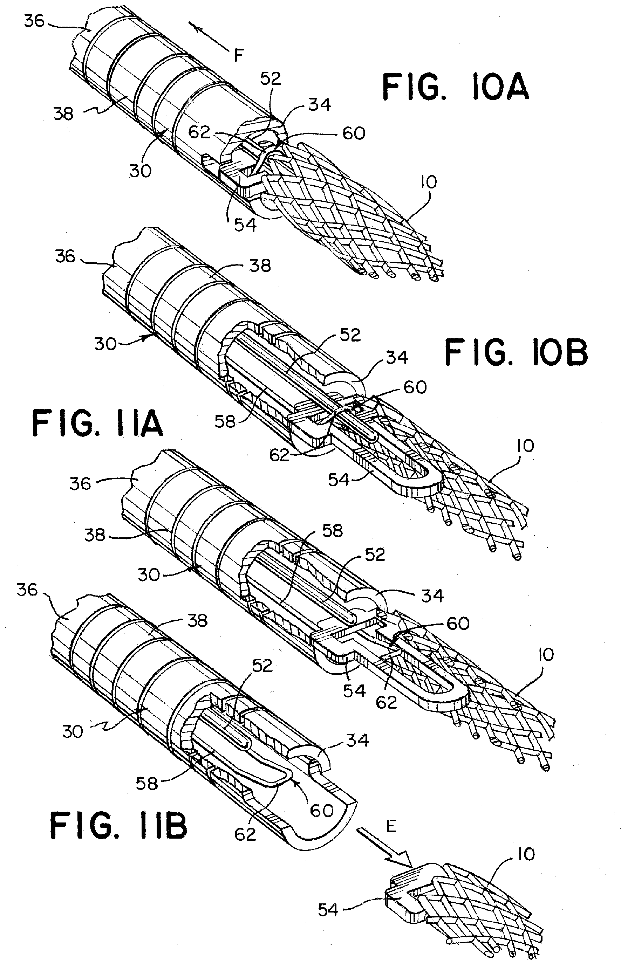

FIGs. 10A through 11B generally illustrate example attachment and delivery between delivery tube 30 and braid 10 for deploying and detaching braid 10 in aneurysm A. FIGs. 10A through 11B is merely one way that delivery tube 30 and braid 10 can be attached at a distal end 34 of the delivery tube 30 and any number of attachment means are contemplated as needed or required. The delivery tube 30 as shown can have a lumen extending from a proximal end 36 to the distal, delivery end 34. FIG. 10A illustrates braid 10 engaged with a locking member 52 and a loop wire 58 locked into the locking portion 54. An opening 60 of the loop wire 58 can be placed through the locking portion 54. The locking portion 54 preferably takes the form of a small diameter elongate filament, however, other forms such as wires or tubular structures are also suitable. While the locking portion 54 is preferably formed of nitinol, other metals and materials such as stainless steel, PTFE, nylon, ceramic or glass fiber and composites may also be suitable. Locking member 52 preferably takes the form of a small diameter elongate filament, however, other forms such as wires or tubular structures are also suitable. While the locking member 52 is preferably formed of nitinol, other metals and materials such as stainless steel, PTFE, nylon, ceramic or glass fiber and composites may also be suitable. When the locking member 52 is put through the opening 60 the braid 10 is now secure. It is understood that delivery tube 30 may include a compressible portion 38 disposed between the distal end of the delivery tube 34 and the proximal end of the delivery tube 36.

The compressible portion 38 can allow the delivery tube 30 to bend and/or flex. Such flexibility can assist tracking the braid 10 through the microcatheter 20 and the tortuous path through the vasculature. The compressible portion 38 can be formed with interference spiral cuts that can allow for gaps to permit bending but in one example, do not act as a spiral-cut spring. Compressible portion 38 can be axially adjustable between an elongated condition and a compressed condition. However, any other arrangement allowing axial adjustment (e.g., a wound wire or spiral ribbon) can also be suitable for use with detachment systems according to the present disclosure). The compressible portion 38 can be in the elongated condition at rest and automatically or resiliently returns to the elongated condition from a compressed condition, unless otherwise constrained. The function of the compressible portion 38 is described in greater detail herein.

As shown in FIG. 10A, force F was previously applied to place the delivery tube 30 in a compressed state. FIG. 10B illustrates the locking member 52 being drawn proximally to begin the release sequence for braid 10. FIG. 11A illustrates the instant the locking member 52 exits the opening 60 and is pulled free of the loop wire 58. The distal end 62 of the loop wire 58 falls away/returns to its preformed shape and exits the locking portion 54. As can be seen, there is now nothing holding the braid 10 to the delivery tube 30. FIG. 11B illustrates the end of the release sequence. Here, the compressible portion 38 of the delivery tube 30 has expanded/returned to its original shape and "sprung" forward. An elastic force E is imparted by the distal end 34 of the delivery tube 30 to the braid 10 to "push" it away to insure a clean separation and delivery of the braid 10 to the aneurysm A. It is to be understood that the delivery scheme described in FIGs. 10A-11B are merely example approaches to delivery of braid 10.

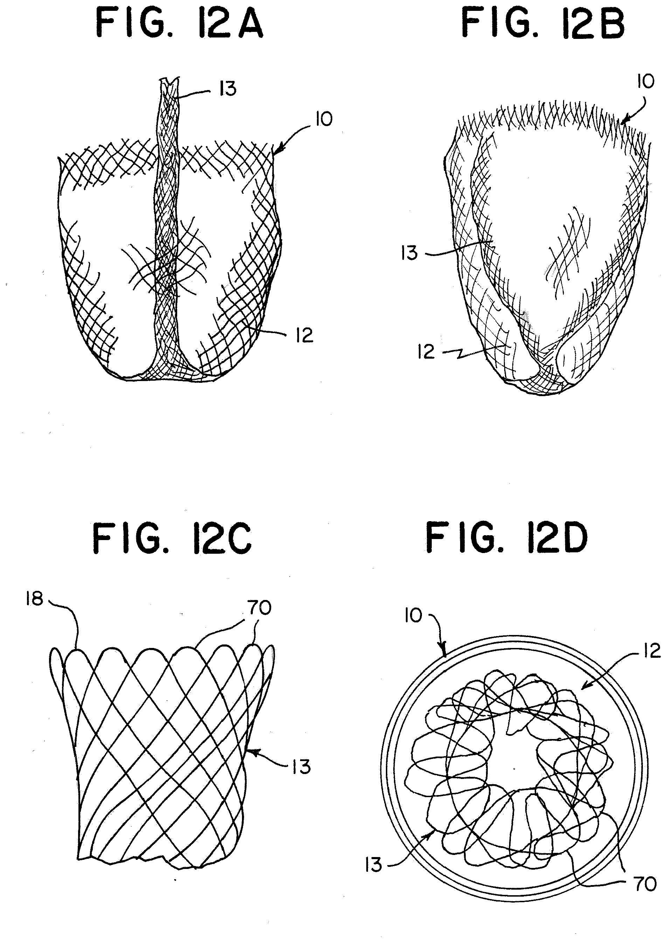

FIGs. 12A through 12D illustrate example prototypes of double layered braids 10. FIGs. 12A through 12D illustrate some varying braid angles, braid patterns, apertures of the mesh, and the like. In FIGs. 12C and 12D, the first open distal end 18 of the second radially expandable segment 13 can include looped ends 70. In one example, the second open distal end 14 can also include looped ends 70. The looped ends 70 can be particularly advantageous to ensure that the braid 10 is atraumatic when in contact with the aneurysm A.

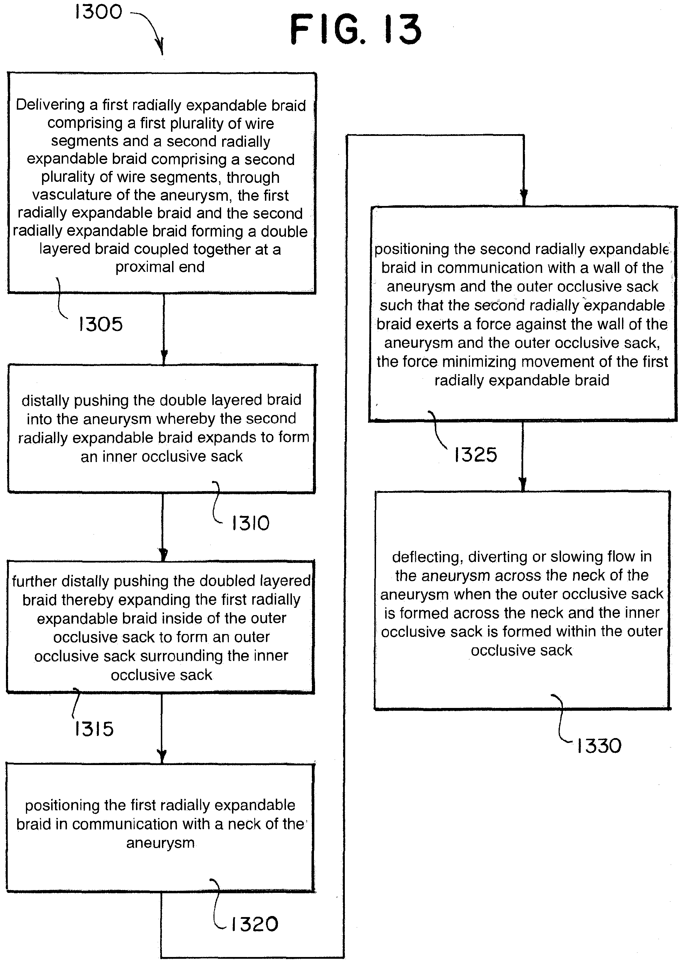

FIG. 13 is a flow diagram outlining the steps of method 1300 for occluding an aneurysm. In step 1305, a first radially expandable braid including a first plurality of wire segments and a second radially expandable braid including a second plurality of wire segments can be delivered through vasculature of the aneurysm. The first radially expandable braid and the second radially expandable braid can form a double layered braid coupled together at a proximal end. A majority of wire segments in the first plurality of wire segments can have a first braid wire diameter that is less than a second braid wire diameter of a majority of the wire segments in the second plurality of wire segments. The first plurality of wire segments can have a first average braid wire diameter and the second plurality of wire segments can have a second average braid wire diameter, the first average braid wire diameter being less than the second average braid wire diameter. Each of the wire segments in the first plurality of wire segments can have a first cross-section area and each of the wire segments in the second plurality of wire segments can have a second cross-section area, the first cross section area being less than the second cross-section area. The braid can be any of the double layered braids as illustrated and described herein, a variation thereof, or an alternative thereto as appreciated and understood by a person having ordinary skill according to the teaching herein.

In step 1310, the double layered braid can be distally pushed into the aneurysm whereby the second radially expandable braid expands to form an inner occlusive sack.

In step 1315, the double layered braid can be further distally pushed into the aneurysm thereby expanding the first radially expandable braid to form the outer occlusive sack surrounding the inner occlusive sack.

In step 1320, the first radially expandable braid can be positioned in communication with a neck of the aneurysm.

In step 1325, the second radially expandable braid can be positioned in communication with a wall of the aneurysm and the outer occlusive sack such that the second radially expandable braid can exert a force against the wall of the aneurysm and the outer occlusive sack. The force can minimize movement of the first radially expandable braid. When the second radially expandable braid is expanding within the outer occlusive sack, the outer occlusive sack can be pushed against the wall of the aneurysm and the neck of the aneurysm.

In step 1330, the flow into the aneurysm across the neck of the aneurysm can be deflected, diverted, or slowed when the outer occlusive sack is formed across the neck and the inner occlusive sack is formed within the outer occlusive sack.

It is understood that variations of the braid 10 can include various materials such as stainless steel, bio absorbable materials, and polymers. Braid 10, including any specific portions such as any breaks, varying regions of differing porosities, and occlusive sacks, can be heat set to various configurations such as spherical, oblong, saddle shaped, or the like, for the purpose of shaping the outer and/or inner sack to better match the aneurysm morphology. In addition, the braid 10 can be heat shaped to include weak points to facility the radial expansion of the occlusive sacks. Further, interstices of braid 10 that form the sacks can vary, or be selectively designed, in size or shape along its length depending on how much braid 10 is caused to radially expand as delivery tube 30 is distally moved.

The specific configurations, choice of materials and the size and shape of various elements can be varied according to particular design specifications or constraints requiring a system or method constructed according to the principles of the disclosed technology. Such changes are intended to be embraced within the scope of the disclosed technology. The presently disclosed embodiments, therefore, are considered in all respects to be illustrative and not restrictive. It will therefore be apparent from the foregoing that while particular forms of the disclosure have been illustrated and described, various modifications can be made without departing from the spirit and scope of the disclosure and all changes that come within the meaning and range of equivalents thereof are intended to be embraced therein.

### Embodiments

1. A method of occluding an aneurysm, comprising:
   delivering a first radially expandable braid comprising a first plurality of wire segments and a second radially expandable braid comprising a second plurality of wire segments, through vasculature to the aneurysm, the first radially expandable braid and the second radially expandable braid forming a double layered braid coupled together at a proximal end;
   distally pushing the double layered braid into the aneurysm whereby the second radially expandable braid expands to form an inner occlusive sack;
   further distally pushing the double layered braid thereby expanding the first radially expandable braid to form an outer occlusive sack surrounding the inner occlusive sack;
   positioning the first radially expandable braid in communication with a neck of the aneurysm;
   positioning the second radially expandable braid in communication with a wall of the aneurysm and the outer occlusive sack such that the second radially expandable braid exerts a force against the wall of the aneurysm and the outer occlusive sack, the force minimizing movement of the first radially expandable braid; and
   deflecting, diverting or slowing flow into the aneurysm across the neck of the aneurysm when the outer occlusive sack is formed across the neck and the inner occlusive sack is formed within the outer occlusive sack.
2. The method of embodiment 1, wherein a majority of wire segments in the first plurality comprise a first braid wire diameter that is less than a second braid wire diameter of a majority of the wire segments in the second plurality of wire segments.
3. The method of embodiment 2, wherein the first plurality of wire segments have a first average braid wire diameter and the second plurality of wire segments have a second average braid wire diameter, the first average braid wire diameter being less than the second average braid wire diameter.
4. The method of embodiment 2, wherein each of the wire segments in the first plurality of wire segments have a first cross-section area and each of the wire segments in the second plurality of wire segments have a second cross-section area, the first cross-section area being less than the second cross-section area.
5. The method of embodiment 2, further comprising:
   expanding the second radially expandable braid within the outer occlusive sack while the outer occlusive sack is pushed against the wall of the aneurysm and the neck of the aneurysm.

## Claims

1. An occlusive device for treating an aneurysm, comprising:
a first radially expandable braided segment configured to form an outer occlusive sack in the aneurysm and comprising a first plurality of wire segments, a majority of wire segments of the first plurality of wire segments respectively comprising a first braid wire diameter; and
a second radially expandable braided segment configured to form an inner occlusive sack in the aneurysm and comprising a second plurality of wire segments, a majority of wire segments of the second plurality of wire segments respectively comprising a second braid wire diameter greater than the first braid wire diameter.

2. The device of claim 1, wherein the first braid wire diameter is between approximately 20 micrometers and approximately 25 micrometers.

3. The device of claim 1 or claim 2, wherein the second braid wire diameter is between approximately 25 micrometers inches and approximately 76 micrometers.

4. The device of any one of the preceding claims, wherein the first plurality of wire segments comprises a greater number of wire segments than the first plurality of wire segments.

5. The device of any one of the preceding claims, wherein, in a deployed configuration, the inner occlusive sack provides a radially outward force against a wall of the aneurysm and the outer occlusive sack.

6. The device of any one of the preceding claims, wherein, in a collapsed configuration, the first expandable braided segment surrounds the second expandable braided segment and the first expandable braided segment and the second expandable braided segment collectively comprise a total wire count and an average wire cross-sectional area, the first expandable braided segment being collapsible to comprise an outer diameter smaller than a single braid comprising a wire count equal to the total wire count and an average wire cross-sectional area equal to the average wire cross-sectional area of the first and second expandable braided segments.

7. The device of any one of the preceding claims, wherein the outer occlusive sack has a first open distal end and the inner occlusive sack has a second open distal end, the second open distal end including a plurality of looped ends.

8. The device of any one of the preceding claims, wherein the first expandable braided segment and the second expandable braided segment are coupled together proximally at a proximal end.

9. The device of any one of the preceding claims, wherein in a deployed configuration, the first expandable braided segment expands radially to a substantially cylindrical first predetermined shape and the second expandable braided segment expands radially to a substantially spherical second predetermined shape.

10. The device of any one of the preceding claims, wherein the first plurality of wire segments has a first average braid wire diameter, the first average braid wire diameter being a sum of the first braid wire diameter of each wire segment of the first plurality of wire segments divided by a total number of wire segments of the first plurality of wire segments.

11. The device of claim 10, wherein the second plurality of wire segments has a second average braid wire diameter, the second average braid wire diameter being a sum of the second braid wire diameter of each wire segment of the second plurality of wire segments divided by a total number of wire segments of the second plurality of wire segments, the second average braid wire diameter being greater than the first average braid wire diameter.

12. The device of claim 11, wherein the first average braid wire diameter is between approximately 20 micrometers and approximately 25 micrometers and the second average braid wire diameter is between approximately 25 micrometers and approximately 76 micrometers.

13. An occlusive device for treating an aneurysm (A), comprising:
a first braid configured to form an outer occlusive sack in the aneurysm and comprising a first plurality of wire segments, a majority of wire segments of the first plurality of wire segments respectively comprising a first cross-section area; and
a second braid configured to form an inner occlusive sack in the aneurysm and comprising a second plurality of wire segments, a majority of wire segments of the second plurality of wire segments respectively comprising a second cross-section area greater than the first cross section area.

14. The device of claim 13, wherein the first cross-section area is between approximately 3.2 × 10⁻⁴ millimeters squared and approximately 5.1 × 10⁻⁴ millimeters squared.

15. The device of claim 13 or claim 14, wherein the second cross-section area is between approximately 5.1 × 10⁻⁴ millimeters squared and approximately 4.6 × 10⁻³ millimeters squared.
